(19) Europäisches Patentamt
European Patent Office
Office européen des brevets

(11) Publication number: **0 130 833 A1**

# (12) EUROPEAN PATENT APPLICATION

(21) Application number: 84304506.3

(22) Date of filing: 29.06.84

(51) Int. Cl.⁴: C 07 D 405/12, A 61 K 31/415, A 61 K 31/44, C 07 D 311/08, C 07 D 213/63, C 07 D 233/56, C 07 D 233/64

(30) Priority: 05.07.83 JP 122222/83

(43) Date of publication of application: 09.01.85 Bulletin 85/2

(84) Designated Contracting States: DE FR GB

(71) Applicant: YAMANOUCHI PHARMACEUTICAL CO. LTD., No. 5-1 Nihonbashi-Honcho, 2-chome Chuo-ku, Tokyo (JP)

(72) Inventor: Kijima, Tadao, No.1086-2 Oaza Sanagaya, Shiraoka-cho (JP)
Inventor: Kageyama, Shunji, No.36-6, Nakano 1-chome, Nakano-ku Tokyo (JP)
Inventor: Okada, Minoru, No.10-20 Higashioi 6-chome, Shinagawa-ku Tokyo (JP)
Inventor: Ohata, Isao, No.47-17, Marugasaki-cho, Omiya-shi Saitama (JP)

(74) Representative: Geering, Keith Edwin, REDDIE & GROSE 16 Theobalds Road, London WC1X 8PL (GB)

(54) **Coumarin compounds, their preparation, and pharmaceutical compositions containing them.**

(57) Coumarin compounds of the formula

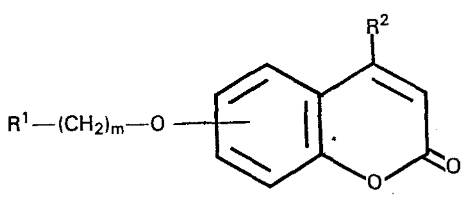

(wherein $R^1$ represents an imidazolyl group or an pyridyloxy group which may be substituted by lower alkyl groups(s); $R^2$ represents a hydrogen atom or a lower alkyl group: and m represents an integer of 1 to 6) and salts thereof inhibit platelet aggregation and are effective for the prophylaxis and the medical treatment of arteriosclerosis, cerebral embolism, cerebral infarction, transient paroxysmal ischemia, angina pectoris, peripheric thrombus and peripheric embolism.

EP 0 130 833 A1

# COUMARIN COMPOUNDS, THEIR PREPARATION, AND PHARMACEUTICAL COMPOSITIONS CONTAINING THEM

This invention relates to the novel coumarin compounds shown by the formula (I), and the salts thereof:

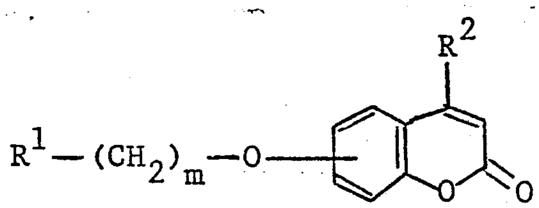

(I)

wherein $R^1$ represents an imidazolyl group or a pyridyloxy group which may be substituted by alkyl group(s); $R^2$ represesnts a hydrogen atom or alkyl group, and m represents an integer of 1 to 6. Any alkyl group will usually be a lower alkyl group.

$R^1$, $R^2$ and m have the above-defined significance throughout.

"Lower alkyl group" as used in this specification includes straight chain and branched chain alkyl groups having from 1 to 5 carbon atoms, such as methyl group, ethyl group, propyl group, isopropyl group, butyl group, isobutyl group, sec-butyl group, tert-butyl group, pentyl group (amyl group), isopentyl group, neopentyl group, tert-pentyl group, etc.

Some typical compounds of this invention are as follows:

7-[5-(1-imidazolyl)pentyloxy]coumarin
7-[5-(6-methyl-3-pyridyloxy)pentyloxy]coumarin
7-[5-(3-pyridyloxy)pentyloxy]coumarin
7-[3-(1-imidazolyl)propoxy]coumarin
7-[5-(6-ethyl-3-pyridyloxy)pentyloxy]coumarin
7-[4-(6-methyl-3-pyridyloxy)butoxy]coumarin
7-[4-(1-imidazolyl)butoxy]coumarin
7-[3-(6-methyl-3-pyridyloxy)propoxy]coumarin
7-[5-(1-imidazolyl)ethoxy]-4-methylcoumarin
7-[(1-imidazolyl)methoxy]coumarin

The compounds of the above formula (I) can form salts. Pharmacologically acceptable such salts include those with inorganic acid such as hydrochloric acid, sulfuric acid, hydrobromic acid, phosphoric acid, etc.; those with an organic acid such as formic acid, acetic

acid, lactic acid, oxalic acid, succninic acid, fumaric acid, maleic acid, methansulfonic acid, ethansulfonic acid, benzenesulfonic acid, etc.; and also the quaternary ammonium salts obtained by reaction with an alkyl halide - e.g. methyl iodide.

The compounds of this invention have platelet aggregation inhibiting activity, and are effective for the prophylaxis and the medical treatment of arteriosclerosis, cerebral embolism, cerebral infarction, transient paroxysmal ischemia, angina pectoris, peripheric thrombus and peripheric embolism.

The pharmacological effects of compounds of this invention were examined in the following experiment.

Platelet aggregation inhibiting activity:

Platelet-rich plasma (PRP) and platelet-poor plasma (PPP) used for the experiment were obtained from Japan white rabbit's venous blood. Platelet aggregation rate and extent were determind in accordance with a method described in Born, G.V.R.; Nature; 194, 927(1962). The effects of compounds of this invention in inhibiting platelet aggregation induced by adding arachidonic acid (final concentration: 0.3 mM) were measured by Paytons aggregometer. The results showed that the compounds of this invention possess inhibiting activity, and that the effective concentration is $IC_{50}$: 10-100 μM.

The compounds of this invention can be formed into various pharmaceutical formulations such as powder, granule, tablet, capsule, injection, etc., using conventional carriers. The compounds can be administered orally (tablet, capsule, granule, powder, liquid, etc.) or parenterally (intravenous injection or intramuscular injection, suppository, etc.). The dosage of the compounds depends upon the condition, age, etc. of the patient, but for oral administration the adult dosage is usually 1-100 (preferably 5-25 mg/kg) a day, which can for example be administered all at once or in 2 to 4 divided doses.

The compounds of this invention can be prepared by various methods, for example, by method shown below:

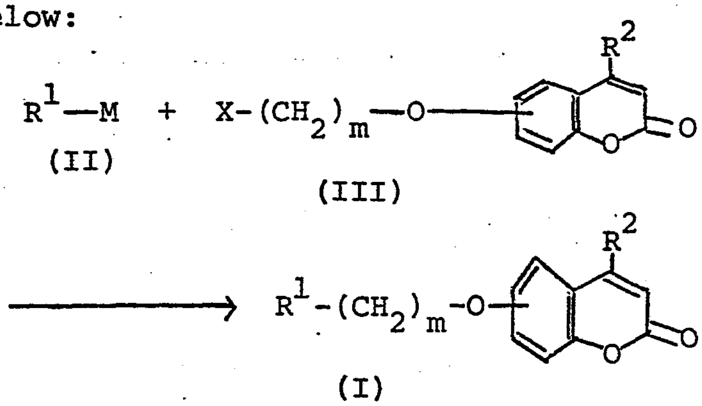

(II) (III)

(I)

wherein M represents a hydrogen atom or alkali metal atom (e.g. Na or K), and X represents a halogen atom (e.g. Cℓ, Br or I); M and X have the above-defined significance throughout this specification.

Thus, a compound of formula (I) can be prepared by reacting an imidazole compound or hydroxypyridine compound (M=H) shown by formula (II) with a halogenoalkyloxycoumarin compound of formula (III) in the presence of a base or a basic material. A compound of formula (I) can be also prepared by reacting an alkali metal salt of said imidazole compound or of said hydroxypyridine compound (M=alkali metal) with a compound of formula (III).

It is preferred that the reaction (that is, N-alkylation or O-alkylation) be carried out at room temperature or under heating in an organic solvent such as alcohol (methanol, ethanol, etc.), dimethylsulfoxide, dimethylformamide, benzene, toluene, xylene, ether, tetrahydrofuran, etc.

Suitable base or basic material for use in the direct reaction between imidazole or hydroxypyridine (II, M=H) and the compound (III) includes potassium carbonate, sodium hydroxide, sodium amide, alkali metal alcoholate such as sodium ethoxide, sodium hydride, etc., and organic lithium compound such as n-butyl lithium, etc.

Other preparation methods

include reacting a halogenoalkyl imidazole or halogenoalkoxy pyridine of the formula (IV)

$$R^1-(CH_2)_m-X$$

with a hydroxycoumarin compound (or the alkali metal salt thereof) of the formula (V)

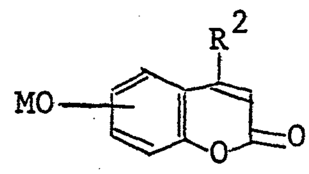

in a manner similar to that described above.

The compounds of this invention prepared as above can be isolated and purified, as the free compounds or the salts thereof, by conventional chemical operations such as recrystallization, extraction, distillation, concentration, various chromatographies, and crystallization, etc.

The present invention is illustrated, without limitation, by the following Examples. Some of the starting compounds of the present invention are novel and preparation of such a compound is shown in the Reference Example. The compound of Reference Example is a compound of formula (III).

Reference Example

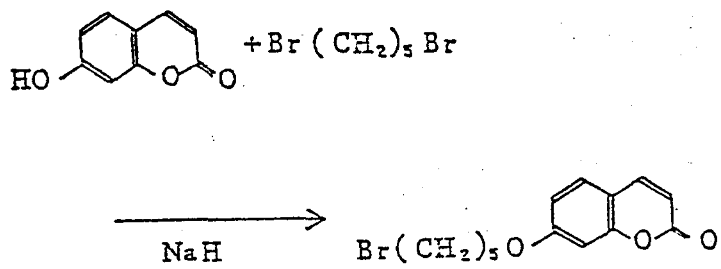


After 4.0 g of sodium hydride (60% suspension in a mineral oil) was washed with dry benzene, 100 ml of dry dimethylformamide was added thereto. While stirring at room temperature, 16.2 g of 7-hydroxycoumarin was added to the mixture. After vigorous bubbling was over, the resulting suspension was heated at 80°C for 30 minutes while stirring and then cooled to room temperature. A solution of 15 ml of 1,5-dibromopentane in 30 ml of dry N,N'-dimethylformamide was added thereto, and heating was conducted at 60°C for 4 hours while stirring. The solvent was removed from the reaction liquid by distillation under reduced pressure. The residue was dissolved in methylene chloride and the solution was successively washed with a 5 % aqueous sodium hydrogen carbonate solution, water and then a saturated aqueous sodium chloride solution followed by drying over anhydrous sodium sulfate. After drying the solvent was distilled off under reduced pressure. The oily residue was subjected to silica gel column chromatography and the product was eluted using a benzene:n-hexane mixture (1 : 5) as

eluant. The solvent was removed from the elute by distillation under reduced pressure to obtain the compound 7-(5-bromopentyloxy)coumarin, as an oily substance.

NMR Spectrum ($CDCl_3$) (internal standard: TMS)

δ (ppm) 1.4—2.2 (m, 6H, $C-(CH_2)_3-C$)

3.46 (t, 2H, $Br-CH_2-C$, J=6.5 Hz)

4.03 (t, 2H, 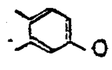 $-CH_2-C$, J=6.5 Hz)

6.24 (d, 1H, 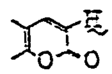 J=10.0 Hz)

6.78 (d, 1H, 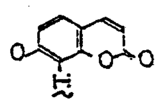 J=1.8 Hz)

6.83 (dd, 1H, 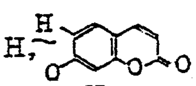 J=9.0 Hz, 1.8 Hz)

7.36 (d, 1H, 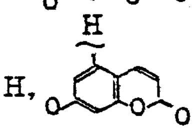 J=9.0 Hz)

7.62 (d, 1H, 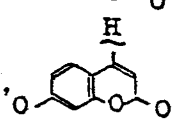 J=10.0 Hz)

Example 1

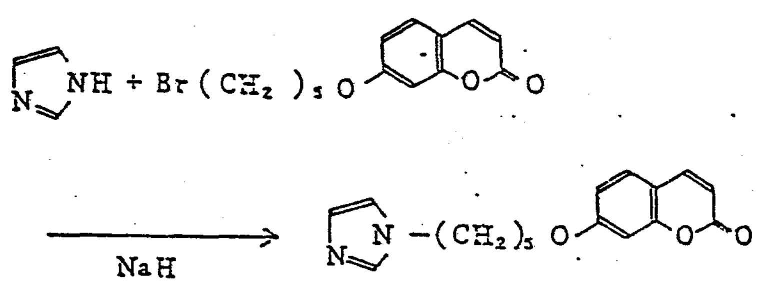

After 0.4 g of sodium hydride (60% suspension in a mineral oil) was washed with dry benzene, 30 ml of dry N,N'-dimethylformamide was added thereto. While stirring at room temperature, 0.7 g of imidazole was added to the mixture. After vigorous bubbling was over, the resulting suspension was heated at 80° C for 30 minutes while stirring and then cooled to room temperature. A solution of 3.1 g of 7-(5-bromopentyloxy)coumarin in 10 ml of dry N,N-dimethylformamide was added thereto, and heating was conducted at 60°C for 4 hours while stirring. The solvent was removed from the reaction liquid by distillation under reduced pressure. The residue was dissolved in methylene chloride and the solution was successively washed with a 5% aqueous sodium hydrogen carbonate solution, water and then a saturated aqueous sodium chloride solution followed by drying over anhydrous sodium sulfate. After drying, the solvent was distilled off under reduced pressure. The residual oily substance was subjected to silica gel column chromatography and the product was eluted using chloroform:methanol (99:1) as an eluant. The solvent was removed from the elute by distillation under reduced pressure to obtain the compound 7-[5-(1-imidazolyl)pentyloxy]coumarin as an oily substance. The hydrochloride salt of this compound has a melting point of 134-135° C after recrystallization from ethanol.

Elemental Analysis (as $C_{17}H_{19}N_2O_3Cl$)

| | C(%) | H(%) | N(%) | Cl(%) |
|---|---|---|---|---|
| Calculated: | 60.99 | 5.72 | 8.37 | 10.59 |
| Found: | 60.73 | 5.88 | 8.36 | 10.30 |

Example 2

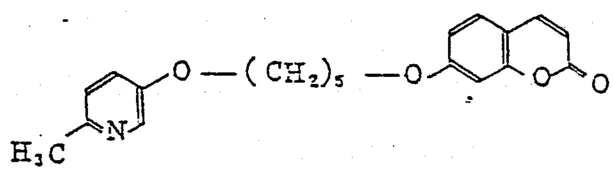

In accordance with the same procedure as in Example 1, using 6-methyl-3-hydroxypyridine instead of imidazole, 7-[5-(6-methyl-3-pyridyloxy)pentyloxy]coumarin was obtained after recrystallization from ethyl acetate.

Melting point: 101-102 °C

Elemental Analysis (as $C_{20}H_{21}NO_4$)

| | C(%) | H(%) | N(%) |
|---|---|---|---|
| Calculated: | 70.78 | 6.24 | 4.13 |
| Found: | 70.51 | 6.35 | 4.09 |

Example 3

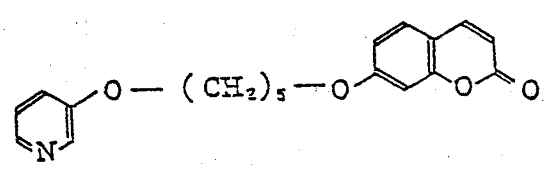

In accordance with the same procedure as in Example 1, using 3-hydroxypyridine instead of imidazole, 7-[5-(3-pyridyloxy)pentyloxy]coumarin was obtained after recrystallization from ethyl acetate.

Melting point: 88-89°C

Elemental Analysis (as $C_{19}H_{19}NO_4$)

| | C(%) | H(%) | N(%) |
|---|---|---|---|
| Calculated: | 70.14 | 5.89 | 4.30 |
| Found: | 70.03 | 5.83 | 4.30 |

C L A I M S :

1. A coumarin compound of the following general formula, or a salt thereof:

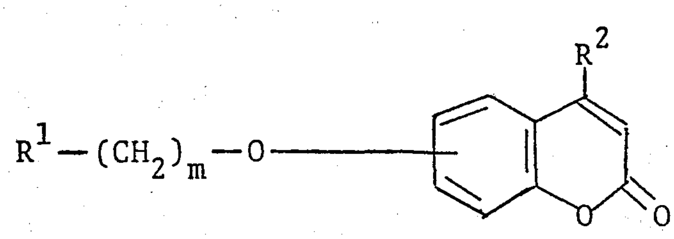

wherein $R^1$ represents an imidazolyl group or an pyridyloxy group which may be substituted by alkyl group(s); $R^2$ represents a hydrogen atom or an alkyl group; and m represents an integer of 1 to 6.

2. A compound according to claim 1 wherein $R^1$ represents 3-pyridyloxy optionally substituted by alkyl.

3. A compound according to claim 1 wherein $R^1$ is 1-imidazolyl.

4. A compound according to claim 1, 2 or 3 wherein the or each said alkyl group contains from 1 to 5 carbon atoms, e.g. is selected from methyl and ethyl groups.

5. A compound according to claim 4 wherein the or each alkyl group is selected from methyl, ethyl, propyl, iso-propyl, butyl, iso-butyl and pentyl groups.

6. One or more compounds selected from the following compounds according to claim 1

7-[-5-(1-imidazolyl)pentyloxy]coumarin;

7-[5-(3-pyridyloxy)pentyloxy]coumarin;

7-[5-(6-methyl-3-pyridyloxy)pentyloxy]coumarin;

7-[5-(3-pyridyloxy)pentyloxy]coumarin; and the salts thereof.

7. A compound according to any preceding claim which is an inorganic or organic acid addition salt, e.g. a hydrochloride.

8. A pharmaceutical composition which comprises a compound according to any preceding claim in a pharmaceutically acceptable carrier.

9. A method of preparing a compound according to claim 1 which comprises (a) reacting a compound $R^1$ — M with a compound

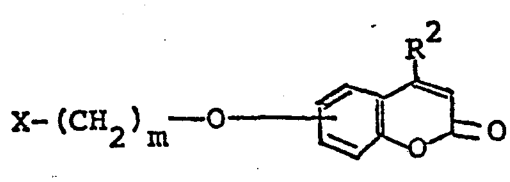

or (b) reacting a compound $R^1-(CH_2)_m-X$ with a compound

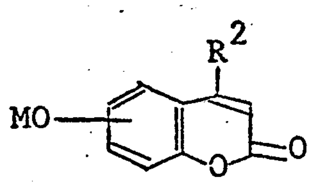

wherein M is a hydrogen or alkali metal atom and X is a halogen atom and $R^1$, $R^2$ and m are as defined in claim 1.

10. A novel compound of formula II, III, IV or V herein.

0130833

European Patent Office

# EUROPEAN SEARCH REPORT

Application number

EP 84304506.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | US - A - 4 330 549 (FRIEBE)<br>* Compounds I,II *<br>-- | 1,10 | C 07 D 405/12<br>A 61 K 31/415<br>A 61 K 31/44<br>C 07 D 311/08<br>C 07 D 213/63<br>C 07 D 233/56<br>C 07 D 233/64 |
| X | DE - B2 - 2 123 924 (BOEHRINGER)<br>* Compounds I,III,IV *<br>-- | 1,10 | |
| A | CHEMICAL ABSTRACTS, vol. 93, no. 11, September 15, 1980, Columbus, Ohio, USA<br>SMITH, AMOS B., III; JERRIS, PAULA J. " Synthesis of geiparvarin: a novel antitumor agent possessing a 3(2H)-furanone ring"<br>page 699, column 2, abstract-no. 114 082z<br>& Tetrahedron Lett.1980, 21(8),711-14<br>-- | 1 | **TECHNICAL FIELDS SEARCHED (Int. Cl. 4)** |
| A | CHEMICAL ABSTRACTS, vol. 90, no. 2, January 8, 1979, Columbus, Ohio, USA<br>JOSEPH, PULLUKAT T; PAVITHRAN, CHORAPPAN; WARRIER, KRISHNAN G.K.; PRABHAKARAN, CHATHAKUDAM P. "Iron(II) complexes of 1-benzyI-2-phenylbenzimidazole and 2-coumarinylbenzimidazole"<br>page 622, column 2, abstract-no. 15 641s<br>& Transition Met. Chem. (Weinheim, Ger.) 1978, 3(5), 286-8<br>-- | 1 | C 07 D 405/00<br>C 07 D 311/00 |

The present search report has been drawn up for all claims

| Place of search | Date of completion of the search | Examiner |
|---|---|---|
| VIENNA | 27-08-1984 | HAMMER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

0130833

European Patent Office

# EUROPEAN SEARCH REPORT

Application number

EP 84304506.3

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 4) |
| X | CHEMICAL ABSTRACTS, vol. 96, no. 21, May 24, 1982, Columbus, Ohio, USA<br>MATSUBARA, TAKASHI; YOSHIHARA, EMIKO; IWATA, TSUYOSHI; TOCHINO, YOSHIHIRO; HACHINO, YOSHIMI "Biotransformation of coumarin derivatives (1) 7-alkoxycoumarin O-dealkylase in liver microsomes"<br>page 13, column 2, abstract-no. 173 844p<br>& Jpn. J. Pharmacol. 1982, 32(1), 9-21 (Eng). The in vitro metabolic fate of 7-alkoxycoumarins<br>---- | 10 | |
| | | | TECHNICAL FIELDS SEARCHED (Int. Cl. 4) |

EPO Form 1503.2 06.78